# EUROPEAN PATENT APPLICATION

(11) **EP 2 229 968 A1**
(43) Date of publication of application: **22.09.2010**
(21) Application number: 09356021.7
(22) Date of filing: 16.03.2009
(51) Int. Cl.: A61M 5/32, A61M 5/50

(54) **Injection device having uncoupling means**

(71) Applicant: Becton Dickinson France, 38800 Le Pont-de-Claix (FR)
(72) Inventor: Dominicy, Eric, 38420 Revel (FR)
(74) Representative: Brédeville, Odile Marie

(57) **Abstract**

The present invention relates to an injection device (100) comprising :
- a container (13) containing a piston (3) attached to a plunger rod (4) provided with a pusher part (5) and a puller part (6) connected by coupling means (30, 31),
- a needle holder (104) capable of engaging said puller part (6) at the end of the injection,
- first retaining means (20, 107, 107a) for maintaining said needle holder (104) in an exposed position,
- first deactivating means (3b, 21) for releasing said first retaining means (20, 107, 107a) at the end of injection,
- uncoupling means (10a) for uncoupling said coupling means (30, 31),

wherein said piston (3) is radially expandable from a stressed state to a rest state and said container (13) has a distal region (10a) with an inner diameter IDp greater than the proximal inner diameter IDs of said container (13), said distal region (10a) enabling the expansion of said piston (3) toward its rest state and forming at least part of said uncoupling means.

## Description

The present invention relates to an injection device provided with a needle, that can be prefilled or filled at the time of injection, and that comprises a mechanism to retract the needle in a controlled manner at the end of the injection.

In this application, the device has a longitudinal axis which is the main axis of the constitutive parts of said device. The distal end of a component or of a device is to be understood as meaning the end furthest from the user's hand and the proximal end is to be understood as meaning the end closest to the user's hand. Likewise, in this application, the "distal direction" is to be understood as meaning the direction of injection, and the "proximal direction" is to be understood as meaning the opposite direction to the direction of injection. The term "inwardly" means towards the device axis, as opposed to the term "outwardly".

An injection device typically comprises a container intended to carry a product to be injected; a needle holder carrying a needle and located within said container; a plunger rod coupled to a piston, on which a user can push in order to move the piston distally, thereby causing the product to be expelled from the container through a distal opening provided with the needle, and realizing the injection of said product.

Preferably, in particular to prevent accidental injury after use of the injection device, the needle should not be exposed after the injection. One solution consists of making the needle manually or automatically retracted inside the injection device container at the end of the injection.

Several known injection devices perform this needle retraction by means of components, which are exterior to the container and which make the device cumbersome, complicated and expensive to design and to assemble. In addition some injection devices require numerous parts to be assembled involving a complicated and costly assembling and manufacturing process. During the use of the injection device, some of these parts are intended to be coupled, uncoupled, connected, disconnected and moved. Depending on their conception, these injection devices may not be enough reliable to fulfill pharmaceutical needs. Therefore, there is a need for injection devices with limited number of parts and enabling reliable use.

Moreover, some injection devices require a specific design of the container, which cannot be obtained with glass containers, due to specific constraints with manufacturing glass parts.

Yet having containers or at least part of containers made of glass is useful for the safe storing of some specific medicine substances, which would otherwise interact, and possibly degrade when in contact with plastic.

Moreover, it is also important that the user be capable of performing the injection with minimum action and effort from his side, so that the injection be safely completed.

Consequently, there is a need for injection devices provided with a system for retracting the needle at the end of the injection, wherein said system would be located within the container, and that would still show a simple design, in particular so that at least part of the container could be made of glass. Besides, there is also a need for such an injection device that can operate automatically and passively, without the need for the user to apply an additional effort or to make a specific gesture, during and at the end of the injection, in particular for neutralizing the needle after use.

The present invention meets these needs by proposing an injection device comprising at least :
- a container, intended to carry a product to be injected, said container comprising an open proximal end and a substantially closed distal end provided with an opening,
- a needle holder carrying a needle and located within said container, said needle holder being movable with respect to said container between an exposed position, in which the distal tip of said needle extends beyond the distal end of said container through said opening, and a retracted position in which the distal tip of said needle does not extend beyond the distal end of said container,
- a piston provided in said container, proximally from said needle holder, said piston being movable with respect to said container, the distal movement of said piston being intended to cause the product to be expelled from said container thereby realizing the injection of said product,
- a plunger rod intended to be coupled to said piston, said plunger rod comprising a puller part and a pusher part, said puller part being capable of engaging at least one of said needle holder or needle at the end of the injection,
- coupling means intended to couple said plunger rod to said piston at least in the distal direction during the injection upon distal pressure exerted on said plunger rod by a user,
- biasing means located between said puller part and said piston and being in one of a stressed or released conditions, said biasing means aiming at moving said puller part proximally with respect to said piston when going from their stressed condition to their released condition,
- first retaining means for maintaining said needle holder in its exposed position before the end of the injection,
- first deactivating means for releasing said first retaining means at the end of the injection,
- uncoupling means for uncoupling said coupling means at the end of the injection, thereby causing said needle holder or needle, engaged with said puller part, to move from its exposed position to its retracted position, upon release of distal pressure exerted on said plunger rod,
   **characterized in that**
   said piston is radially expandable from a stressed state to a rest state,
   said container comprises a distal region with an inner diameter IDp greater than the proximal inner diameter IDs of said container, said distal region enabling the expansion of said piston toward its rest state and forming at least part of said uncoupling means.

Thanks to this arrangement, the invention makes it possible to provide injection devices with a system for retracting the needle at the end of the injection integrated with the container. In particular, the injection device is provided with a limited number of parts and a reliable mechanism enabling the efficient uncoupling of the pusher and puller part to allow safe needle retraction. Moreover, the injection device of the invention makes it possible to use glass barrels for at least part of the container. It is therefore possible to store specific medicine substances that would otherwise degrade if stored in plastic barrels.

Moreover, the injection device of the invention can be operated automatically and passively, without the need for the user to apply an additional effort or to make a specific gesture, during and at the end of the injection, in particular for neutralizing the needle after use. In particular, the device shows a simple design.

In an embodiment of the injection device of the invention, said first retaining means include at least a movable part, movable between a lock position when said needle holder is in its exposed position and an unlock position to allow said needle holder to move toward its retracted position.

Said movable part may be located within said distal region.

In embodiments, of the injection device of the invention further comprises second retaining means for maintaining said puller part and said pusher part attached to each other at least in translation up to the end of injection, and second deactivating means for releasing said second retaining means at the end of the injection.

In embodiments of the injection device of the invention, said second retaining means include a retaining surface located either on said puller part or on said pusher part, and a locking element, located respectively either on said pusher part or on said puller part, said locking element being engaged in said retaining surface before the end of the injection, said locking element being intended to abut against flanges provided at the proximal end of said container at the end of the injection to disengage said locking element from said retaining surface, said flanges and said locking element forming at least part of said second deactivating means.

In embodiments of the injection device of the invention, said piston is traversed by a central axial hole in which is received a puller part distal part, said coupling means include at least a shape provided on said piston or on said puller part outer surface and a complementary shape respectively provided on said puller part distal part or on said piston, said shape and complementary shape being able to cooperate with each other so as to maintain said puller part coupled to said piston up to the end of the injection.

In an embodiment of the injection device of the invention, said piston is provided with an annular rib and said puller part distal part is provided with an annular groove, said annular rib being locked in said annular groove up to the end of the injection.

In an embodiment of the injection device of the invention said container comprises a proximal part including a sleeve open at both ends, and a distal part mounted on the distal end of said sleeve and closing substantially the distal end of said container, said distal part being provided with said opening and with said distal region.

In another embodiment, the injection device of the invention comprises a rib and a groove, one being provided at the distal end of said sleeve the other being provided at the proximal end of said proximal part, said sleeve being attached to said plug part by means of said rib being engaged within said groove. For example, a seal may be provided at the junction between said rim and said groove.

In embodiments of the injection device of the invention, said sleeve is made out of glass. Such an embodiment allows storing specific medicines that would otherwise degrade on contact with plastics. Thanks to the specific structure of the device, for which the container combines a sleeve part and plug part, the manufacture of part of the container in glass, in particular the sleeve part, is facilitated since the global shape of the sleeve is tubular, tubular parts being simple to shape from glass material.

The injection device of the invention will now be further described in reference to the following description and attached drawings in which:
Fig. 1 is a sectional view of a first embodiment of the injection device before injection,
Fig. 2 is a sectional view of the injection device of figure 1 at the end of the injection just prior to retraction of the needle,
Fig. 3 is a sectional view of the injection device of figures 1 and 2 after retraction of the needle.

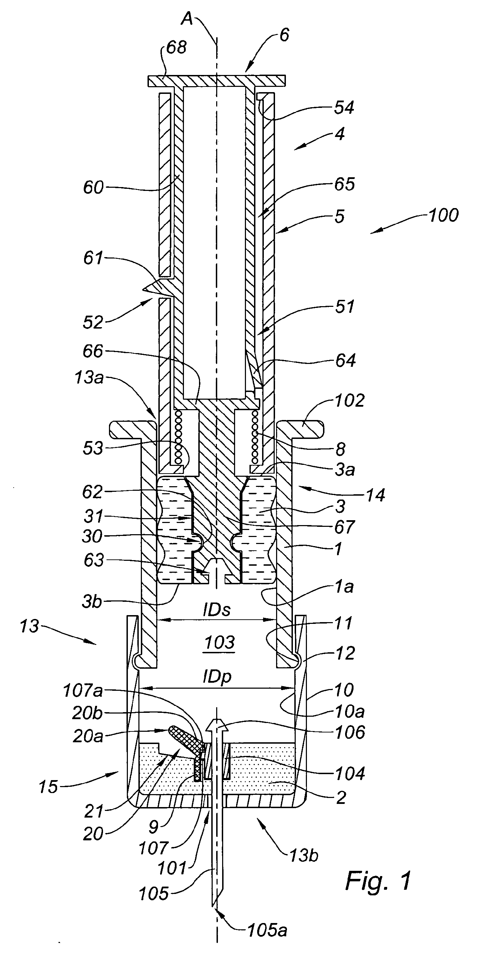

With reference to Figure 1, is shown a longitudinal cross section of an injection device according to a first embodiment of the present invention and generally designated by reference number 100.

The injection device 100 has a longitudinal axis A and comprises a container 13, intended to carry a product to be injected. The container 13 comprises an open proximal end 13a and a substantially closed distal end 13b provided with an opening 101. The container 13 comprises a proximal part 14 formed on the example shown by a sleeve (or barrel) 1 open at both ends and which may be made of glass or plastic. In particular, when the sleeve 1 is made of glass, its manufacture is very simple since the shape of the sleeve 1 is globally tubular. The sleeve 1 is provided at its proximal end with flanges 102 extending outwardly: such flanges 102 constitute bearing surfaces for the fingers of the user at the time of injection. The container 13 further comprises a distal part 15, mounted at the distal end of the sleeve 1 and closing substantially the distal end 13b of the container 13. The opening 101 is therefore provided on said distal part 15. The distal part 15 includes a plug part 10 which realizes the junction between the proximal part 14 and the distal part 15 of the container 13. On the example shown, the plug part 10 is integrated with the distal part 15. The plug part 10 is provided on its inner wall 10a with an inner annular groove 12 and the sleeve 1 is provided on its outer wall with an external annular rib 11, the plug part 10, and thus the distal part 15, being attached to the sleeve 1 through the external annular rib 11 being engaged in said inner annular groove 12. The plug part 10 has, at least on a certain region defined by its inner wall 10a, an inner diameter IDp greater than the inner diameter IDs of the sleeve 1. In another embodiment not shown, the distal part 15 and the proximal part 14 are attached to each other by any suitable means such as for example gluing, friction force.

The injection device 100 also comprises a needle seal (or first stopper) 2 and a piston (or second stopper) 3, both housed in the container 13, and which may both be made out of elastomeric material. The needle seal 2 is located at the distal end 13b of the container 13, ie at the distal end of the distal part 15 in the example shown. The needle seal 2 is locked in translation for example through ribs (not shown) provided at the distal end of the distal part 15, for example at the distal end of the plug part 10.

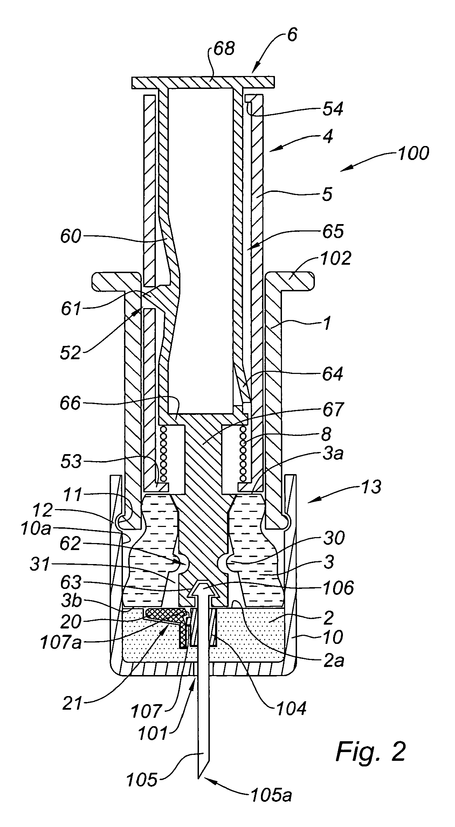

The needle seal 2 and the piston 3 are in seal tight contact respectively with the inner walls 1 a, 10a of the sleeve 1 and of the plug part 10. The piston 3 is located proximally further from the needle seal 2 in order to define, with said needle seal 2 and with the inner walls 1 a, 10a of both the sleeve 1 and the plug part 10, a storing space 103 in which the product to be injected is stored. The piston 3 is able to move distally in regards to the sleeve 1 and to the plug part 10 from a "before injection" position (see Fig. 1), where the piston 3 is located within the inner wall 1 a of the sleeve 1, toward an end of injection" position (see Fig. 2) where the piston 3 is substantially located within the inner wall 10a of the plug part 10. The distal movement of the piston 3 is therefore intended to cause the product to be expelled from the container 13 thereby realizing the injection of said product. The piston 3 is traversed by a central axial hole 31 and is provided on its internal surface with an annular rib 30.

As will appear from the description below, the piston 3 is expandable from a stressed state, when it is located in the sleeve 1, to a rest state, when it is substantially located in the plug part 10, where it has more space to expand laterally, due to the inner diameter IDp of the inner wall 10a of the plug part 10 being greater than the inner diameter IDs of the inner wall 1a of the sleeve 1. In its rest state in the inner wall 10a of the plug part 10, the piston 3 is still in seal tight contact with the inner wall 10a of the plug part 10 and it is able to ensure tightness and prevent any leakage of the product.

The injection device 100 of figures 1-3 further comprises a needle 105 linked to a needle holder 104 at least partly embedded in the needle seal 2. The needle holder 104 is provided with an outer rib 107 defining a proximal abutment surface 107a. The proximal extremity of the needle 105 extends inside the storing space 103, and it is provided with a hook 106, the function of which will be explained later. The needle 105 goes through the opening 101 to allow the product to be expelled from the storing space 103.

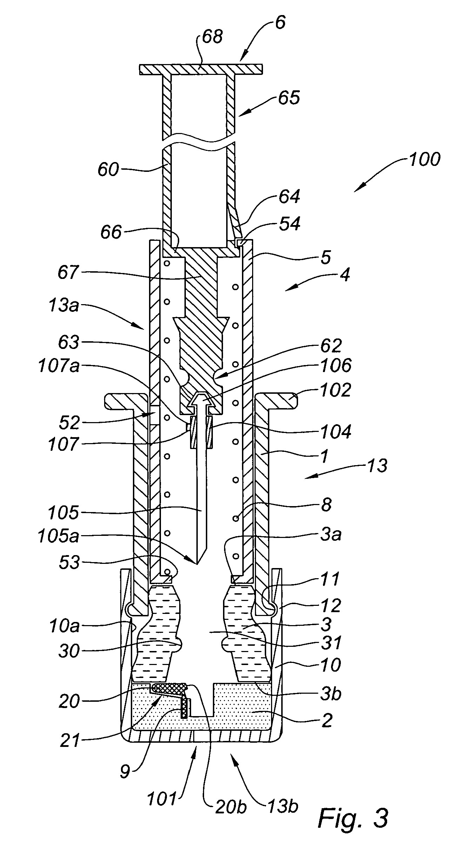

The needle seal 2 comprises on its proximal surface an intermediary part, under the form in the example shown of a rod 9 provided with a movable part, such as a deflecting leg 20 : the rod 9 is fixedly embedded within the needle seal 2 and therefore forms an immobile part with respect to the plug part 10. The deflecting leg 20 is an elastically deformable element and is able to move from a lock position (see Fig. 1) in which it is in a released state and in which it locks the needle holder 104 in the needle seal 2 to an unlock position (see Fig. 2 and 3) in which the deflecting leg 20 is in a stressed state and in which the needle holder 104 is free to move in regards to the needle seal 2. The deflecting leg 20 comprises an outward part 20a and an inward part 20b. The needle seal 2 is further provided on its proximal surface with a recess 21.

In the lock position of the deflecting leg 20, as shown on figure 1, the outward part 20a of the deflecting leg 20 extends outwardly and proximally inside space 103, and the inward part 20b of the deflecting leg 20 is in abutment against the proximal abutment surface 107a of the outer rib 107 of the needle holder 104, thereby preventing any movement of said needle holder 104 with respect to the needle seal 2. In the unlock position of the deflecting leg 20, as shown on figures 2 and 3 and as will appear from the description below, the deflecting leg 20 is deflected under distal pressure exerted on its outward part 20a by a distal surface 3b of the piston 3 coming directly in contact with said deflecting leg 20: as a consequence, the outward part 20a of the deflecting leg 20 is deflected in the recess 21 provided in the needle seal 2 and the inward part 20b is disengaged from the abutment surface 107a of the rib 107 of the needle holder 104. The needle seal 2 and the deflecting leg 20 can be made of one piece or of several parts linked to each other by any suitable means such as for example glue or friction.

The injection device 100 of figures 1-3 further comprises a plunger rod 4 made of two main parts, a pusher part 5 and a puller part 6. In the example shown, the pusher part 5 defines a housing 51 having a general tubular shape, in which the puller part 6 is partially located. A locking hole 52 is provided on the longitudinal wall of the housing 51 of the pusher part 5. The housing 51 is further provided at its distal end with an annular inner rim 53, and at its proximal end with an inner radial stop 54.

The puller part 6 comprises a tubular element 65 comprising at least a longitudinal wall 60 able to be elastically deformed and provided on its outer face with a tooth 61 having a triangular shape and extending outwardly. The tubular element 65 is provided in its distal region with a deflecting part 64 extending distally and outwardly. The tubular element 65 is closed at its distal end by a distal transversal wall 66 and is then prolonged in the distal direction by a distal shaft 67 which forms a distal part of the puller part 6 and which is embedded in the piston 3. The outer surface of the shaft 67 is provided with an annular groove 62 cooperating with the annular rib 30 to couple the puller part 6 to the piston 3 before and during the injection, thereby acting as coupling means to couple the plunger rod 4 to the piston at least in the distal direction upon distal pressure exerted on said plunger rod 4 by a user. The distal extremity of the shaft 67 of the puller part 6 is further provided with an inner catching shape 63 complementary to the shape of the hook 106 of the needle 105. The tubular element 65 is closed at its proximal end by a proximal transversal wall 68, located outside from the housing 51 of the pusher part 5, and defining a pushing surface for the user to exert a distal pressure on the plunger rod 4.

Before the injection, the tooth 61 is engaged within the locking hole 52 provided on the longitudinal wall of the pusher part 5. The locking hole 52 forms a retaining surface for said tooth 61 which acts as a locking element. In this position, the annular rib 30 is also locked in the annular groove 62 provided on the outer surface of the shaft 67 of the puller part 6.

The pusher part 5 and the puller part 6 are coupled to each other by biasing means, such as a helical spring 8 tending to urge them in opposite directions. The helical spring 8 is located between the puller part 6 and the piston 3, namely between the shaft 67 of the puller part 6 and the distal region of the housing 51 of the pusher part 5. As appears from figure 1, the proximal end of the helical spring 8 bears on the distal surface of the distal transversal wall 66 and the distal end of the helical spring 8 bears on the proximal surface on the annular inner rim 53. During the storage and the injection position, displacement of the pusher part 5 in regard to the puller part 6 is prevented by the tooth 61 being engaged in the locking hole 52. The distal displacement of the pusher part 5 in regard to the puller part 6 is also prevented by the abutment of the annular inner rim 53 against the piston 3.

During the injection, the proximal extremity of the puller part 6, ie the proximal transversal wall 68, abuts against the proximal extremity, among which the inner radial stop 54, of the pusher part 5. Therefore, distal displacement of the puller part 6, in particular upon distal pressure exerted by the user on the proximal transversal wall 68, leads to the distal displacement of the pusher part 5.

In another embodiment not shown, the tubular element comprises a rigid longitudinal wall provided on its outer face with a tooth elastically deformable forming a locking element enable to lock the puller part in regards to the pusher part. The locking element can also comprise any other suitable means able to prevent the displacement of the puller part in regards to the pusher part before the end of the injection and free the proximal displacement of the puller part in regards to the pusher part at the end of the injection. The locking element can also be provided on the pusher part.

The injection device 100 can be prefilled or filled at the time of the injection. In case of prefilled injection device 100, the "before injection" position corresponds to the storage position of the injection device 100. In case the injection device 100 is filled at the time of the injection, the piston 3 is first located at the distal end inside the sleeve 1 without the catching shape 63 reaching the hook 106. There is therefore a free space between the needle seal 2 and the stopper 3, this free space containing air that will be purged by the user before performing the injection. To fill the injection device 100, the user pulls back the plunger rod 4 to suck the product to be injected in the space 103 formed by the distance created between the needle seal 2 and the piston 3. Because the pusher part 5 and the puller part 6 are locked to each other by means of tooth 61 being locked in locking hole 52, and by the distal and proximal abutment of the pusher part 5 against respectively piston 3 and the puller part 6, both parts (5, 6) move in the proximal direction. In both configurations of the injection device 100, a vein test can be performed before injecting the product by slightly pulling back the plunger rod 4, moving distally the pusher 5, the puller 6 and the piston 3.

The tightness of the product stored in the injection device 100 is performed by the needle seal 2, the piston 3 and optionally by an additional seal (not shown) provided in the area of the annular rib 11 of the sleeve 1 and the annular groove 12 of the plug part 10. The distal end 13b of the container 13 can receive a needle cover (not shown) to protect the needle 105 and prevent needle stick before use.

The operation of the injection device 100 of the invention to inject a product will now be explained with reference to figures 1-3.

The user is provided with the injection device 100 in the position as shown on figure 1. As already mentioned, in this position, the needle holder 104 is locked in the needle seal 2, by means of deflecting leg 20 and outer rib 107 and proximal abutment surface 107a acting as first retaining means. In this position also, the helical spring 8 is maintained in a stressed state, ie a compressed state in the example shown, by means of the tooth 61 being engaged in the locking hole 52, acting as second retaining means. The puller 6 of the plunger rod 4 is linked to the piston 3 by means of annular rib 30 of piston 3 being engaged in annular groove 62 of shaft 67 and by means of inner wall 1a of sleeve 1 maintaining piston 3 in a stressed state. The distal end 105a of the needle 105 extends beyond the distal end 13b of the container 13.

To use the prefilled injection device 100, the user holds the injection device 100 by the flanges 102 applies the injection device 100 on the injection site (not shown), inserts the needle 105 and exerts a distal force on the proximal extremity of the plunger rod 4, ie on the proximal transversal wall 68 of the puller part 6. Under this distal force, the plunger rod 4 as a whole (pusher part 5 and puller part 6) moves distally and displaces the piston 3 towards the needle seal 2, causing the storing space 103 to be reduced and causing the expulsion of the contained product out of the injection device 100 through the needle 105.

As shown on Fig. 2, toward the end of the injection, the distal movement of the tooth 61 causes said tooth 61 to abut against the flanges 102 and the inner wall 1 a of the sleeve 1. The triangular shape of the tooth 61 causes the longitudinal wall 60 to be elastically deformed inwardly. The tooth 61 is radially moved within the sleeve 1 and is disengaged from the locking hole 52. The flanges 102 and the locking hole 52 therefore act as second deactivating means for releasing the second retaining means. In the meantime the piston 3 is displaced distally. As appears clearly on figure 2, a substantial part of the piston 3 comes out from the sleeve 1 and expands in the plug part 10, thanks to the inner diameter IDp of the region 10a of the plug part 10 being greater than the inner diameter IDs of the sleeve 1. This causes the annular rib 30 of the piston 3 to be disengaged from the annular groove 62 provided on the shaft 67 of the puller part 6. The puller part 6 is then free to move proximally. The region 10a delimited by the inner diameter IDp of the plug part 10 therefore acts as uncoupling means for uncoupling the coupling means. In the meantime, the catching shape 63 of the shaft 67 of the puller part 6 has engaged the hook 106 provided at the proximal extremity of the needle 105, thereby connecting the needle 105 to the puller part 6. In the meantime also, the distal surface 3b of the piston 3 has put a distal pressure on the outward part 20a of the deflecting leg 20 and has therefore caused said deflecting leg 20 to deflect from its lock position to its unlock position. The needle holder 104 is then free to move proximally. The distal surface 3b of the piston 3 and the recess 21 therefore act as first deactivating means for releasing the first retaining means. At this stage, the puller part 6 is still under the pressure of the user's thumb preventing any proximal displacement and retraction of the needle 105.

As shown on Figure 3, upon release of the pressure of the user's thumb on the plunger rod 4, the helical spring 8 tends to come back to its natural released condition and it urges proximally the puller part 6 that pulls back the needle 105 in the injection device 100. The puller part 6 is distally locked in the retracted position by the deflecting part 64 abutting against the proximal inner radial stop 54 of the pusher part 5. The needle 105 is then fully retracted in the pusher part 5 and its distal end 105a does not extend beyond the distal end 13b of the container 13. During all the retraction, the thumb of the user is on the proximal extremity of the plunger rod 4 via the proximal transversal wall 68. Therefore, the user is able to control the rate of the retraction of the needle 105 inside the injection device 100.

In another embodiment not shown, the deflecting part provided on the distal region of the tubular element can be replaced by any other suitable means enabling to lock the puller part in regards to the pusher part in its retracted position.

After the retraction, the plunger rod 4, which encompasses the needle 105, can be taken out from the sleeve 1 to be thrown away without any risk of needle stick. The fact that the plunger rod 4 can be thrown away separately from the sleeve 1 presents the advantage of reducing the size of the wastes.

As appears clearly from this description, the injection device 100 is a passively activated one, there is no additional effort to be applied by the user to activate the retraction. In addition, as during retraction there is no stopper to be displaced, there is less friction to be overcome by the helical spring 8.

The injection device of the invention shows advantages over the devices of the prior art. In particular, the injection device of the invention has a system for retracting the needle at the end of the injection, which is integrated in the container. In particular, the device shows a simple design, which makes it possible to use glass barrels for at least part of the container. It is therefore possible to store specific medicine substances that would otherwise degrade if stored in plastic barrels.

Moreover, the injection device of the invention can be operated automatically and passively, without the need for the user to apply an additional effort or to make a specific gesture, during and at the end of injection, in particular for neutralizing the needle after use.

## Claims

1. Injection device (100) comprising at least :
- a container (13), intended to carry a product to be injected, said container (13) comprising an open proximal end (13a) and a substantially closed distal end (13b) provided with an opening (101),
- a needle holder (104) carrying a needle (105) and located within said container (13), said needle holder (104) being movable with respect to said container (13) between an exposed position, in which the distal tip (105a) of said needle (105) extends beyond the distal end (13b) of said container (13) through said opening (101), and a retracted position in which the distal tip (105a) of said needle (105) does not extend beyond the distal end (13b) of said container (13),
- a piston (3) provided in said container (13), proximally from said needle holder (104), said piston (3) being movable with respect to said container (13), the distal movement of said piston (3) being intended to cause the product to be expelled from said container (13) thereby realizing the injection of said product,
- a plunger rod (4) intended to be coupled to said piston (3), said plunger rod (4) comprising a puller part (6) and a pusher part (5), said puller part (6) being capable of engaging at least one of said needle holder (104) or needle (105) at the end of the injection,
- coupling means (30, 62) intended to couple said plunger rod (4) to said piston (3) at least in the distal direction during the injection upon distal pressure exerted on said plunger rod (4) by a user,
- biasing means (8) located between said puller part (6) and said piston (3) and being in one of a stressed or released conditions, said biasing means (8) aiming at moving said puller part (6) proximally with respect to said piston (3) when going from their stressed condition to their released condition,
- first retaining means (20, 107, 107a) for maintaining said needle holder (104) in its exposed position before the end of the injection,
- first deactivating means (3b, 21) for releasing said first retaining means (20, 107, 107a) at the end of the injection,
- uncoupling means (10a) for uncoupling said coupling means (30, 62) at the end of the injection, thereby causing said needle holder (104) or needle (105), engaged with said puller part (6), to move from its exposed position to its retracted position, upon release of distal pressure exerted on said plunger rod (4),
**characterized in that**
said piston (3) is radially expandable from a stressed state to a rest state,
said container (13) comprises a distal region (10a) with an inner diameter IDp greater than the proximal inner diameter IDs of said container (13), said distal region (10a) enabling the expansion of said piston (3) toward its rest state and forming at least part of said uncoupling means.

2. Injection device (100) according to claim 1, wherein said first retaining means include at least a movable part (20), movable between a lock position when said needle holder (104) is in its exposed position, and an unlock position to allow said needle holder (104) to move toward its retracted position.

3. Injection device (100) according to claim 2, wherein said movable part (20) is located within said distal region (10a).

4. Injection device (100) according to any one of claim 1 and 2 wherein it further comprises second retaining means (52, 61) for maintaining said puller part (6) and said pusher part (5) attached to each other at least in translation up to the end of the injection, and second deactivating means (52, 102) for releasing said second retaining means (52) at the end of the injection.

5. Injection device (100) according to claim 4, wherein said second retaining means include a retaining surface (52) located either on said puller part (6) or on said pusher part (5), and a locking element (61), located respectively either on said pusher (5) part or on said puller part (6), said locking element (61) being engaged in said retaining surface (52) before the end of the injection, said locking element (61) being intended to abut against flanges (102) provided at the proximal end (13a) of said container (13) at the end of the injection to disengage said locking element (61) from said retaining surface (52), said flanges (102) and said locking element forming at least part of said second deactivating means (52, 102).

6. Injection device (100) according to any one of claims 1 to 5, wherein said piston (3) is traversed by a central axial hole (31) in which is received a puller part distal part (67), said coupling means include at least a shape (30, 62) provided on said piston (3) or on said puller part distal part (67) and a complementary shape (62, 30, ) respectively provided on said puller part distal part (67) or on said piston (3), said shape (30, 62) and complementary shape (30, 62) being able to cooperate with each other so as to maintain said puller part (6) coupled to said piston (3) up to the end of the injection.

7. Injection device (100) according to claim 6, wherein said piston (3) is provided with an annular rib (30) and said puller part distal part (67) is provided with an annular groove (62), said annular rib (30) being locked in said annular groove (62) up to the end of the injection.

8. Injection device (100) according to claim any one of claims 1 to 7, wherein said container (13) comprises a proximal part (14) including a sleeve (1) open at both ends, and a distal part (15) mounted on the distal end of said sleeve (1) and closing substantially the distal end (13b) of said container (13), said distal part (15) being provided with said opening (101) and with said distal region (10a).

9. Injection device (100) according to claim 8, wherein it further comprises a rib (11) and a groove (12), one being provided at the distal end of said sleeve (1) the other being provided at the proximal end of said proximal part (14), said sleeve (1) being attached to said plug part (10) by means of said rib (11) being engaged within said groove (12).

10. Injection device (100) according to claim 9, wherein a seal is provided at the junction between said rib (11) and said groove (12).

11. Injection device (100) according to any one of claims 8 to 10, wherein said sleeve (1) is made out of glass.
